# EUROPÄISCHE PATENTSCHRIFT

(11) **EP 2 477 701 B1**
(45) Veröffentlichungstag und Bekanntmachung des Hinweises auf die Patenterteilung: **08.03.2017**
(21) Anmeldenummer: 10737037.1
(22) Anmeldetag: 23.07.2010
(51) Int. Cl.: A61K 8/41, A61Q 5/10, A61Q 5/08

(54) **SCHONENDE FÄRBE- UND AUFHELLMITTEL MIT VERBESSERTER AUFHELLLEISTUNG**
GENTLE DYEING AND LIGHTENING AGENTS HAVING IMPROVED LIGHTENING POWER
PRODUIT DE COLORATION ET D'ÉCLAIRCISSEMENT DOUX AYANT UNE PERFORMANCE D'ÉCLAIRCISSEMENT AMÉLIORÉE

(30) Priorität: 17.09.2009 DE 102009029548
(43) Veröffentlichungstag der Anmeldung: 25.07.2012
(73) Patentinhaber: Henkel AG & Co. KGaA, 40589 Düsseldorf (DE)
(72) Erfinder: METTEN, Diane, 40229 Düsseldorf (DE); FRANKE, Ina, 40211 Düsseldorf (DE); WESER, Gabriele, 41472 Neuss (DE); SCHÖPGENS, Jürgen, 41366 Schwalmtal (DE)
(86) Internationale Anmeldenummer: PCT/EP2010/060705
(87) Internationale Veröffentlichungsnummer: WO 2011/032762

(56) Entgegenhaltungen:
- WO-A2-2009/080670
- FR-A1- 2 922 760

## Beschreibung

Die vorliegende Erfindung betrifft Mittel zur oxidativen Aufhellung und/oder Färbung keratinischer Fasern, insbesondere menschlichen Haaren, enthaltend mindestens zwei strukturell unterschiedliche Alkanolamine und mindestens ein Oxidationsmittel, wobei die Mittel frei von Ammoniak sind, sowie deren Verwendung zur Verbesserung der Aufhellleistung.

Die Veränderung von Form und Farbe der Haare stellt einen wichtigen Bereich der modernen Kosmetik dar. Zur modischen Farbgestaltung von Frisuren oder zur Kaschierung von ergrautem oder gar weißem Haar mit modischen oder natürlichen Farbtönen greift der Verbraucher zu aufhellenden oder farbverändernden Mitteln. Diese Mittel sollen neben der gewünschten Färbe- und Aufhellleistung möglichst minimale Schädigungen auf dem Haar hervorrufen und die Faserstruktur schonen.

Zur Bereitstellung farbverändernder kosmetischer Mittel, insbesondere für keratinhaltige Fasern, wie beispielsweise menschliche Haare, kennt der Fachmann je nach Anforderungen an die Färbung diverse Färbesysteme. Für permanente, intensive Färbungen mit entsprechenden Echtheitseigenschaften werden sogenannte Oxidationsfärbemittel verwendet, bei denen die eigentlichen Farbstoffe unter dem Einfluss von Oxidationsmitteln oder von Luftsauerstoff aus den sogenannten Oxidationsfarbstoffvorprodukten gebildet werden. Die Oxidationsfärbemittel zeichnen sich zwar durch hervorragende, lang anhaltende Färbeergebnisse aus. Für temporäre Färbungen werden üblicherweise Färbe- oder Tönungsmittel verwendet, die als färbende Komponente sogenannte direktziehende Farbstoffe enthalten. Hierbei handelt es sich um Farbstoffmoleküle, die direkt auf das Substrat aufziehen und keinen oxidativen Prozess zur Ausbildung der Farbe benötigen.

Zur Aufhellung bzw. zum Blondieren menschlicher Haare werden üblicherweise Oxidationsmittelzubereitungen wie Wasserstoffperoxid-Lösung eingesetzt. Diese Aufhellung kann mit oxidativen Färbevorgängen bzw. Färbungen mit direktziehenden Farbstoffen kombiniert werden.

Insbesondere oxidative Aufhell- und Haarfärbemittel sind trotz ihrer vorteilhaften Färbeeigenschaften für den Anwender mit Nachteilen behaftet. Vor ihrer Anwendung auf menschliches Haar werden oxidative Färbemittel üblicherweise mit verdünnter wässriger Wasserstoffperoxid-Lösung vermischt. Diese Mischung wird dann auf das Haar aufgebracht und nach einer bestimmten Einwirkzeit wieder ausgespült. Der Einsatz dieser Oxidationsmittel zur Ausfärbung respektive Entwicklung der eigentlichen Färbung führt zu Schädigungen in der Haarstruktur und auf der Haaroberfläche. Eine Verbesserung der Färbeleistung eines Mittels hinsichtlich Farbintensität würde eine Reduktion von Anwendungsmenge oder Anwendungsdauer erlauben.
Zudem laufen oxidative Färbe- und Aufhellprozesse an keratinischen Fasern üblicherweise bei alkalischen pH-Werten ab, insbesondere zwischen 9,0 und 10,5. Diese pH-Werte sind notwendig, um eine Öffnung der äußeren Schuppenschicht (Cuticula) zu gewährleisten und eine Penetration der aktiven Spezies (Farbstoffvorprodukte und/oder Wasserstoffperoxid) in das Haar zu ermöglichen. Als Alkalisierungsmittel wird üblicherweise Ammoniak eingesetzt, der allerdings für den Anwender den Nachteil des intensiven Geruches und eventueller Reizung bis hin zu Hautirritationen und Hautsensibilisierungen aufweist.

In der Schrift WO 2009/080670 A2 werden Haarbehandlungsmittel beschrieben, die Alkanolamine und Aminosäuren enthalten. In einer Beispielformulierung der WO 2009/080670 A2 werden Monoethanolamin und 2-Amino-2-methylpropan-1-ol im Gewichtsverhältnis 1:3 eingesetzt.

Auch wenn die bislang auf dem Markt befindlichen Aufhell- und Färbemittel in der Regel gute Färbeleistungen zeigen, so können sie aufgrund von Haarschädigung, langen Anwendungszeiten und der aufgrund der hohen Konzentrationen an Oxidations- und Alkalisierungsmittel möglichen Hautreizungen nicht als optimal angesehen werden.
Aufgabe der vorliegenden Erfindung ist es daher, die Echtheitseigenschaften, die Grauabdeckungsleistung und Aufhellleistung der Färbungen von ammoniakfreien Haarfärbe-/-aufhellmitteln soweit zu verbessern, dass sie mit den üblichen, sich auf dem Markt befindlichen, ammoniak-haltigen Mitteln vergleichbar oder ihnen überlegen sind, gleichzeitig jedoch eine verringerte Haarschädigung aufweisen.
Es wurde nun in nicht vorhersehbarer Weise gefunden, dass durch den Einsatz bestimmter Alkanolamine als Alkalisierungsmittel anstelle von Ammoniak zu farbverändernden, oxidativen Mitteln eine signifikante Verbesserung der Aufhellleistung an keratinischen Fasern erzielt werden kann. Auch verfügen oxidative Haarfärbemittel mit dieser Alkalisierung über eine verbesserte Aufhellleistung.
Der Einsatz von Monoethanolamin oder 2-Amino-2-methylpropanol in der Haarfärbung ist dem Fachmann seit langem bekannt bekannt. Aus dem Stand der Technik ist bislang nicht ersichtlich, dass durch die spezielle Kombination zweier Alkanolamine die Leistung der Mittel bei gleichem pH-Wert in ammoniakfreien Färbemitteln unter Erhalt hervorragender Aufhell- und Färbeleistungen eingesetzt werden können.

Ein erster Gegenstand der vorliegenden Erfindung ist daher ein Mittel zur oxidativen Färbung und/oder Aufhellung keratinischer Fasern, insbesondere menschlicher Haare, enthaltend in einem kosmetischen Träger mindestens ein Oxidationsmittel, ausgewählt aus Wasserstoffperoxid und/oder dessen feste Anlagerungsprodukte an anorganische oder organische Verbindungen, mindestens ein Alkanolamin gemäß Formel (I),

NH₃₋ₓ(CH₂CH₂OH)ₓ (I),

worin x für eine der Zahlen 1, 2 oder 3 steht,
und mindestens ein Alkanolamin gemäß Formel (II), 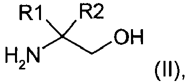 worin R1 und R2 jeweils unabhängig voneinander für Wasserstoff, eine C₁-C₆-Alkylgruppe oder eine C₁-C₆-Hydroxyalkylgruppe stehen oder R1 und R2 zusammen mit dem Kohlenstoffatom des Alkanolamins einen Ring bilden, welcher 4 bis 8 Ringatome besitzt und gegebenenfalls 1 bis 2 Heteroatome enthalten kann, mit der Massgabe, dass R1 und R2 nicht gleichzeitig für Wasserstoff stehen,
welches dadurch gekennzeichnet ist, dass das Mittel frei von Ammoniak ist und dass das Mittel Alkanolamine gemäß Formel (I) und Alkanolamine gemäß Formel (II) in einer Gesamtmenge von 0,5 bis 25 Gew.-%, bezogen auf das Gesamtgewicht des anwendungsbereiten Mittels, enthält, wobei das Gewichtsverhältnis zwischen Alkanolaminen gemäß Formel (I) und Alkanolaminen gemäß Formel (II) einen Wert von 1 zu 2 bis 2 zu 1 besitzt.

Unter keratinischen Fasern oder auch Keratinfasern sind dabei Pelze, Wolle, Federn und insbesondere menschliche Haare zu verstehen. Obwohl die erfindungsgemäßen Mittel in erster Linie zum Färben von Keratinfasern geeignet sind, steht prinzipiell einer Verwendung auch auf anderen Gebieten nichts entgegen.

Die zur erfindungsgemäßen Verwendung eingesetzten Zubereitungen enthalten die Wirkstoffe in einem kosmetischen Träger. Dieser kosmetische Träger ist im Sinne der Erfindung wässrig, alkoholisch oder wässrig-alkoholisch. Unter wässrig-alkoholischen Trägern sind im Sinne der vorliegenden Erfindung wasserhaltige Zusammensetzungen, enthaltend 3 bis 70 Gew.-% eines C₁-C₄-Alkohols, bezogen auf das Gesamtgewicht der Anwendungsmischung, insbesondere Ethanol bzw. Isopropanol, zu verstehen. Die erfindungsgemäßen Mittel können zusätzlich weitere organische Lösungsmittel, wie beispielsweise 4-Methoxybutanol, Ethyldiglykol, 1,2-Propylenglykol, n-Propanol, n-Butanol, n-Butylenglykol, Glycerin, Diethylenglykolmonoethylether, und Diethylenglykolmono-n-butylether, enthalten. Bevorzugt sind dabei alle wasserlöslichen organischen Lösungsmittel. Ein wässriger Träger enthält im Sinne der Erfindung mindestens 30 Gew.-%, insbesondere mindestens 50 Gew.-% Wasser, bezogen auf das Gesamtgewicht der Anwendungsmischung. Zum Zwecke der Haarfärbung sind solche Träger beispielsweise Cremes, Emulsionen, Gele oder auch tensidhaltige schäumende Lösungen, wie beispielsweise Shampoos, Schaumaerosole oder andere Zubereitungen, die für die Anwendung auf dem Haar geeignet sind. Bevorzugte Träger stellen dabei Emulsionen und Gele dar, wobei Emulsionen besonders bevorzugt sind.

Die Begriffe "frei von Ammoniak" bzw. "ammoniakarm" und "ammoniakfrei" im Sinne der Erfindung beziehen sich dabei auf die dem Mittel zugesetzte Ammoniakmenge, wobei der Ammoniakzusatz sowohl als wässrige, alkoholische, wässrig-alkoholische oder anderweitige Lösung als auch durch Ammoniakgaseinleitung oder Zusatz von verflüssigtem Ammoniak erfolgen kann. Der Zusatz von Ammoniak kann aber auch durch Verwendung von entsprechenden Ammonium-Salzen erfolgen, wobei das Ammonium-Kation je nach pH-Wert der Zubereitung im Gleichgewicht mit seiner korrespondierenden Base, dem Ammoniak selbst, steht. Die Begriffe "ammoniakfrei" bzw. "ammoniakarm" im Sinne der Erfindung beziehen sich daher auch auf Mittel, die Ammonium-Salze enthalten.
Der Begriff "frei von Ammoniak" bedeutet dabei, dass das anwendungsbereite Mittel weniger als 2 Gew.-% zugesetzten Ammoniak, bezogen auf das Gesamtgewicht der Anwendungszubereitung, enthält. Sofern Ammonium-Salze im anwendungsbereite Mittel vorhanden sind, so beträgt der Ammoniakanteil, der sich aus diesen Salzen unter Annahme einer vollständigen Deprotonierung der Ammonium-Kationen ergibt, entsprechend weniger als 2 Gew.-%, bezogen auf das Gesamtgewicht der Anwendungszubereitung. Bevorzugte, ammoniakarme Mittel enthalten weniger als 1 Gew.-%, insbesondere weniger als 0,5 Gew.-% und ganz besonders bevorzugt weniger als 0,1 Gew.-% zugesetzten Ammoniak, jeweils bezogen auf das Gesamtgewicht der Anwendungszubereitung. Ammoniakfrei im Sinne der Erfindung sind solche Mittel, denen kein Ammoniak durch eine der oben beschriebenen Methoden zugesetzt wurde. Solche Mittel sind erfindungsgemäß besonders bevorzugt.
Als ersten wesentlichen Inhaltsstoff enthält die erfindungsgemäße Zubereitung mindestens ein Alkanolamin gemäß Formel (I), NH₃₋ₓ(CH₂CH₂OH)ₓ (I), worin x für eine der Zahlen 1, 2 oder 3 steht. Erfindungsgemäß geeignete Alkanolamine der Formel (I) sind Monoethanolamin (2-Aminoethanol; (x = 1)), Diethanolamin (Bis-(2-hydroxyethyl)amin; (x = 2)) und Triethanolamin (Tris-(2-hydroxyethyl)amin; (x = 3)). Besonders bevorzugte Mittel sind dadurch gekennzeichnet, dass sie als Alkanolamin gemäß Formel (I) Monoethanolamin enthalten.
In einer Ausführungsform der vorliegenden Erfindung enthalten die Mittel ein Alkanolamin gemäß Formel (I) zu 0,5 bis 15 Gew.-%, bevorzugt zu 1 bis 10 Gew.-% und besonders bevorzugt zu 2 bis 6 Gew.-%, jeweils bezogen auf das anwendungsbereite Mittel.
Als weiteren wesentlichen Inhaltsstoff enthält die erfindungsgemäße Zubereitung mindestens ein Alkanolamin gemäß Formel (II), 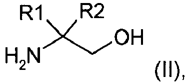 worin R1 und R2 jeweils unabhängig voneinander für Wasserstoff, eine C₁-C₆-Alkylgruppe oder eine C₁-C₆-Hydroxyalkylgruppe stehen oder R1 und R2 zusammen mit dem Kohlenstoffatom des Alkanolamins einen Ring bilden, welcher 4 bis 8 Ringatome besitzt und gegebenenfalls 1 bis 2 Heteroatome enthalten kann, mit der Massgabe, dass R1 und R2 nicht gleichzeitig für Wasserstoff stehen.

Beispiele für C₁-C₆-Alkylreste sind die Gruppen -CH₃, -CH₂CH₃, -CH₂CH₂CH₃, -CH(CH₃)₂, -CH₂CH₂CH₂CH₃, -CH₂CH(CH₃)₂, -CH(CH₃)CH₂CH₃, -C(CH₃)₃.
Beispiele für eine C₁-C₆-Hydroxyalkylgruppe sind -CH₂OH, -CH₂CH₂OH, -CH₂CH₂CH₂OH, -CH₂CH(OH)CH₃ und -CH₂CH₂CH₂CH₂OH, wobei die Gruppe -CH₂CH₂OH bevorzugt ist. Beispiele für Verbindungen, bei denen R1 und R2 zusammen mit dem Kohlenstoffatom des Alkanolamins einen Ring bilden, welcher 4 bis 8 Ringatome besitzt und gegebenenfalls 1 bis 2 Heteroatome enthalten kann, sind 1-Amino-1-hydroxymethylcyclooctan ,1-Amino-1-hydroxymethylcycloheptan 1-Amino-1-hydroxymethylcyclohexan, 1-Amino-1-hydroxymethyl-cyclopentan, 1-Amino-1-hydroxymethylcyclobutan, 4-Amino-4-hydroxymethyl-piperidin, 3-Amino-3-hydroxymethylpiperidin, 4-Amino-4-hydroxymethyl-tetrahydropyran, 3-Amino-3-hydroxymethyl-tetrahydropyran, 3-Amino-3-hydroxymethyl-pyrrolidin, 3-Amino-3-hydroxy-methyl-tetrahydrofuran.

Eine Ausführungsform ist dadurch gekennzeichnet, dass das Mittel als Alkanolamin gemäß Formel (II) mindestens eine Verbindung gemäß Formel (II) enthält, worin R1 und/oder R2 für eine C₁-C₆-Alkylgruppe stehen oder R1 und R2 gemeinsam mit dem Kohlenstoffatom des Alkanolamins für einen Cyclopentyl-Rest, einen Cyclohexyl-Rest oder einen Tetrahydropyran-Rest stehen.

Besonders bevorzugt stehen R1 und R2 jeweils für eine Methylgruppe.

In einer Ausführungsform der vorliegenden Erfindung enthalten die Mittel ein Alkanolamin gemäß Formel (II) zu 0,5 bis 15 Gew.-%, bevorzugt zu 1 bis 10 Gew.-% und besonders bevorzugt zu 2 bis 6 Gew.-%, jeweils bezogen auf das anwendungsbereite Mittel.

Eine bevorzugte Ausführungsform der vorliegenden Erfindung ist dadurch gekennzeichnet, dass das erfindungsgemäße Mittel als Alkanolamin gemäß Formel (I) und als Alkanolamin gemäß Formel (II) mindestens eine Verbindung gemäß Formel (II) enthält, worin R1 und R2 jeweils für eine Methylgruppe stehen.

Erfingsungsgemäße Mittel sind dadurch gekennzeichnet, dass das Mittel Alkanolamine gemäß Formel (I) und Alkanolamine gemäß Formel (II) in einer Gesamtmenge von 0,5 bis 25 Gew.-%, bevorzugt von 1 bis 20 Gew.-% und besonders bevorzugt von 4 bis 12 Gew.-%, jeweils bezogen auf das Gesamtgewicht des anwendungsbereiten Mittels, enthält, wobei das Gewichtsverhältnis zwischen Alkanolaminen gemäß Formel (I) und Alkanolaminen gemäß Formel (II) einen Wert von 1 zu 2 bis 2 zu 1, besitzt.

Bevorzugte Mittel enthalten gleiche Gewichtsanteile an Alkanolaminen gemäß Formel (I) und Alkanolaminen gemäß Formel (II).

Als dritten wesentlichen Inhaltsstoff enthält das Mittel als Oxidationsmittel mindestens ein Oxidationsmittel, ausgewählt aus Wasserstoffperoxid und seinen Anlagerungsverbindungen an feste anorganische oder organische Verbindungen, enthält.

Bevorzugt wird Wasserstoffperoxid selbst als wässrige Lösung verwendet. Wasserstoffperoxid kann aber auch in Form einer festen Anlagerungsverbindung von Wasserstoffperoxid an anorganische oder organische Verbindungen, wie beispielsweise Natriumperborat, Natriumpercarbonat, Magnesiumpercarbonat, Natriumpercarbamid, Polyvinylpyrrolidinon n H₂O₂ (n ist eine positive ganze Zahl größer 0), Harnstoffperoxid und Melaminperoxid, eingesetzt werden. Im letztgenannten Fall setzen die Anlagerungsverbindungen in der erfindungsgemäßen Anwendungsmischung Wasserstoffperoxid frei. Diese Mittel enthalten neben der Anlagerungsverbindung im kosmetischen Träger freies Wasserstoffperoxid.

Erfindungsgemäß ganz besonders bevorzugt wird das Wasserstoffperoxid dem erfindungsgemäßen Mittel als wässrige Wasserstoffperoxid-Lösung zudosiert. Die Konzentration einer Wasserstoffperoxid-Lösung wird einerseits von den gesetzlichen Vorgaben und andererseits von dem gewünschten Effekt bestimmt; vorzugsweise werden 6 bis 25 Gew.-%ige Lösungen in Wasser verwendet. Erfindungsgemäß bevorzugte Mittel sind dadurch gekennzeichnet, dass sie - bezogen auf ihr Gesamtgewicht - 0,01 bis 25 Gew.-%, vorzugsweise 0,1 bis 15 Gew.-%, besonders bevorzugt 1 bis 12 Gew.-% Wasserstoffperoxid (berechnet als 100%iges H₂O₂) enthalten.

In einer bevorzugten Ausführungsform enthalten die erfindungsgemäßen Aufhell- und Färbemittel mindestens eine farbverändernde Komponente. Die farbverändernde Komponente wird dabei ausgewählt aus mindestens einem Oxidationsfarbstoffvorprodukt und/oder direktziehenden Farbstoff und/oder naturanalogen Farbstoff.

In einer Ausführungsform der vorliegenden Erfindung enthält das Mittel als farbverändernde Komponente mindestens ein Oxidationsfarbstoffvorprodukt und/oder direktziehenden Farbstoff. In einer bevorzugten Ausführungsform der erfindungsgemäßen Verwendung enthält die Färbezubereitung als farbverändernde Komponente mindestens ein Oxidationsfarbstoffvorprodukt.
Als Oxidationsfarbstoffvorprodukte enthalten die Färbezubereitungen mindestens eine Entwicklerkomponente und gegebenenfalls mindestens eine Kupplerkomponente. Die Entwicklerkomponenten können untereinander, bevorzugt aber mit Kupplerkomponenten die eigentlichen Farbstoffe ausbilden. Vorzugsweise enthalten die erfindungsgemäßen Färbemittel daher mindestens ein Oxidationsfarbstoffvorprodukt vom Entwicklertyp und mindestens ein Oxidationsfarbstoffvorprodukt vom Kupplertyp. Die Entwickler- und Kupplerkomponenten werden üblicherweise in freier Form eingesetzt. Bei Substanzen mit Aminogruppen kann es aber bevorzugt sein, sie in Salzform, insbesondere in Form der Hydrochloride und Hydrobromide oder der Sulfate einzusetzen.

Dabei werden Entwicklerkomponenten und Kupplerkomponenten im Allgemeinen in etwa molaren Mengen zueinander eingesetzt. Wenn sich auch der molare Einsatz als zweckmäßig erwiesen hat, so ist ein gewisser Überschuss einzelner Oxidationsfarbstoffvorprodukte nicht nachteilig, so dass Entwicklerkomponenten und Kupplerkomponenten in einem Mol-Verhältnis von 1 : 0,5 bis 1 : 2 enthalten sein können.

Geeignete Oxidationsfarbstoffvorprodukte vom Entwicklertyp sind p-Phenylendiamin und dessen Derivate. Bevorzugte p-Phenylendiamine werden ausgewählt aus einer oder mehrerer Verbindungen der Gruppe, die gebildet wird aus p-Phenylendiamin, p-Toluylendiamin, 2-Chlor-p-phenylendiamin, 2,3-Dimethyl-p-phenylendiamin, 2,6-Dimethyl-p-phenylendiamin, 2,6-Diethyl-p-phenylendiamin, 2,5-Dimethyl-p-phenylendiamin, N,N-Dimethyl-p-phenylendiamin, N,N-Diethyl-p-phenylendiamin, N,N-Dipropyl-p-phenylendiamin, 4-Amino-3-methyl-(N,N-diethyl)anilin, N,N-Bis-(2-hydroxyethyl)-p-phenylendiamin, 4-N,N-Bis-(2-hydroxyethyl)-amino-2-methylanilin, 4-N,N-Bis-(2-hydroxyethyl)-amino-2-chloranilin, 2-(2-Hydroxyethyl)-p-phenylendiamin, 2-(1,2-Dihydroxyethyl)-p-phenylendiamin, 2-Fluor-p-phenylendiamin, 2-Isopropyl-p-phenylendiamin, N-(2-Hydroxypropyl)-p-phenylendiamin, 2-Hydroxymethyl-p-phenylendiamin, N,N-Dimethyl-3-methyl-p-phenylendiamin, N-Ethyl-N-2-hydroxyethyl-p-phenylendiamin, N-(2,3-Dihydroxypropyl)-p-phenylendiamin, N-(4'-Aminophenyl)-p-phenylendiamin, N-Phenyl-p-phenylendiamin, 2-(2-Hydroxyethyloxy)-p-phenylendiamin, 2-Methoxymethyl-p-phenylendiamin, 2-(2-Acetylaminoethyloxy)-p-phenylendiamin, N-(2-Methoxyethyl)-p-phenylendiamin, N-(4-Amino-3-methylphenyl)-N-[3-(1H-imidazol-1-yl)propyl]amin, 5,8-Di-aminobenzo-1,4-dioxan sowie ihren physiologisch verträglichen Salzen. Erfindungsgemäß besonders bevorzugte p-Phenylendiaminderivate sind ausgewählt aus mindestens einer Verbindung der Gruppe p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, N-(4-Amino-3-methylphenyl)-N-[3-(1 H-imidazol-1-yl)propyl]amin, 2-Methoxymethyl-p-phenylendiamin sowie den physiologisch verträglichen Salzen dieser Verbindungen.

Es kann erfindungsgemäß weiterhin bevorzugt sein, als Entwicklerkomponente Verbindungen einzusetzen, die mindestens zwei aromatische Kerne enthalten, die mit Amino- und/oder Hydroxylgruppen substituiert sind. Bevorzugte zweikernige Entwicklerkomponenten werden insbesondere ausgewählt aus mindestens einer Verbindung der Gruppe, gebildet aus N,N'-Bis-(2-hydroxyethyl)-N,N'-bis-(4'-aminophenyl)-1,3-diaminopropan-2-ol, N,N'-Bis-(2-hydroxyethyl)-N,N'-bis-(4'-aminophenyl)ethylendiamin, N,N'-Bis-(4'-aminophenyl)tetramethylendiamin, N,N'-Bis-(2-hydroxyethyl)-N,N'-bis-(4'-aminophenyl)-tetramethylendiamin, N,N'-Bis-(4-(methyl-amino)phenyl)tetramethylendiamin, N,N'-Diethyl-N,N'-bis-(4'-amino-3'-methylphenyl)ethylendiamin, Bis-(2-hydroxy-5-aminophenyl)methan, N,N'-Bis-(4'-aminophenyl)-1,4-diazacycloheptan, N,N'-Bis-(2-hydroxy-5-aminobenzyl)piperazin, N-(4'-Aminophenyl)-p-phenylendiamin und 1,10-Bis-(2',5'-diaminophenyl)-1,4,7,10-tetraoxadecan sowie ihre physiologisch verträglichen Salze. Besonders bevorzugte zweikernige Entwicklerkomponenten werden ausgewählt unter N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan oder eines deren physiologisch verträglichen Salze.

Weiterhin kann es erfindungsgemäß bevorzugt sein, als Entwicklerkomponente ein p-Aminophenolderivat oder eines seiner physiologisch verträglichen Salze einzusetzen. Bevorzugte p-Aminophenole sind p-Aminophenol, N-Methyl-p-aminophenol, 4-Amino-3-methyl-phenol, 4-Amino-3-fluorphenol, 2-Hydroxymethylamino-4-aminophenol, 4-Amino-3-hydroxymethylphenol, 4-Amino-2-(2-hydroxyethoxy)-phenol, 4-Amino-2-methylphenol, 4-Amino-2-hydroxymethylphenol, 4-Amino-2-methoxymethyl-phenol, 4-Amino-2-aminomethylphenol, 4-Amino-2-(2-hydroxyethyl-aminomethyl)phenol, 4-Amino-2-(1,2-dihydroxyethyl)phenol, 4-Amino-2-fluorphenol, 4-Amino-2-chlorphenol, 4-Amino-2,6-dichlorphenol, 4-Amino-2-(diethylaminomethyl)-phenol sowie ihre physiologisch verträglichen Salze. Besonders bevorzugte Verbindungen sind p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol.

Ferner kann die Entwicklerkomponente ausgewählt sein aus o-Aminophenol und seinen Derivaten, wie beispielsweise 2-Amino-4-methylphenol, 2-Amino-5-methylphenol oder 2-Amino-4-chlorphenol.

Weiterhin kann die Entwicklerkomponente ausgewählt sein aus heterocyclischen Entwicklerkomponenten, wie beispielsweise aus Pyrimidin-Derivaten, Pyrazol-Derivaten, Pyrazolopyrimidin-Derivaten bzw. ihren physiologisch verträglichen Salzen. Bevorzugte Pyrimidin-Derivate sind insbesondere die Verbindungen 2,4,5,6-Tetraaminopyrimidin, 4-Hydroxy-2,5,6-triaminopyrimidin, 2-Hydroxy-4,5,6-triaminopyrimidin, 2-Dimethylamino-4,5,6-triaminopyrimidin, 2,4-Dihydroxy-5,6-diaminopyrimidin und 2,5,6-Triaminopyrimidin. Bevorzugte Pyrazol-Derivate sind die Verbindungen, die ausgewählt werden unter 4,5-Diamino-1-methylpyrazol, 4,5-Diamino-1-(2-hydroxyethyl)pyrazol, 3,4-Diaminopyrazol, 4,5-Diamino-1-(4'-chlorbenzyl)pyrazol, 4,5-Diamino-1,3-dimethylpyrazol, 4,5-Diamino-3-methyl-1-phenylpyrazol, 4,5-Diamino-1-methyl-3-phenylpyrazol, 4-Amino-1,3-dimethyl-5-hydrazinopyrazol, 1-Benzyl-4,5-diamino-3-methylpyrazol, 4,5-Diamino-3-t-butyl-1-methylpyrazol, 4,5-Diamino-1-t-butyl-3-methylpyrazol, 4,5-Diamino-1-(2-hydroxyethyl)-3-methylpyrazol, 4,5-Diamino-1-ethyl-3-methylpyrazol, 4,5-Diamino-1-ethyl-3-(4-methoxyphenyl)pyrazol, 4,5-Diamino-1-ethyl-3-hydroxymethylpyrazol, 4,5-Diamino-3-hydroxymethyl-1-methylpyrazol, 4,5-Diamino-3-hydroxymethyl-1-isopropylpyrazol, 4,5-Diamino-3-methyl-1-isopropylpyrazol, 4-Amino-5-(2-aminoethyl)amino-1,3-dimethylpyrazol, sowie deren physiologisch verträglichen Salze, insbesondere jedoch 4,5-Diamino-1-(2-hydroxyethyl)pyrazol. Als Pyrazolopyrimidine sind insbesondere Pyrazolo[1,5-a]pyrimidine bevorzugt, wobei bevorzugte Pyrazolo[1,5-a]pyrimidine ausgewählt sind aus Pyrazolo[1,5-aJpyrimidin-3,7-diamin, 2,5-Dimethyl-pyrazolo[1,5-a]pyrimidin-3,7-diamin, Pyrazolo[1,5-a]pyrimidin-3,5-diamin, 2,7-Dimethylpyrazolo[1,5-a]-pyrimidin-3,5-diamin, 3-Aminopyrazolo[1,5-a]pyrimidin-7-ol, 3-Aminopyrazolo[1,5-a]pyrimidin-5-ol, 2-(3-Aminopyrazolo[1,5-a]pyrimidin-7-ylamino)ethanol, 2-(7-Aminopyrazolo[1,5-a]-pyrimidin-3-ylamino)ethanol, 2-[(3-Aminopyrazolo[1,5-a]pyrimidin-7-yl)-(2-hydroxyethyl)-amino]ethanol, 2-[(7-Aminopyrazolo[1,5-a]pyrimidin-3-yl)-(2-hydroxyethyl)amino]ethanol, 5,6-Dimethylpyrazolo[1,5-a]pyrimidin-3,7-diamin, 2,6-Dimethylpyrazolo[1,5-a]pyrimidin-3,7-diamin, 3-Amino-7-dimethylamino-2,5-dimethylpyrazolo[1,5-a]pyrimidin sowie ihre physiologisch verträglichen Salze und ihre tautomeren Formen.

Besonders bevorzugte Entwicklerkomponenten werden ausgewählt aus mindestens einer Verbindung aus der Gruppe, die gebildet wird aus p-Phenylendiamin, p-Toluylendiamin, 2-(2-Hydroxyethyl)-p-phenylendiamin, 2-(1,2-Dihydroxyethyl)-p-phenylendiamin, N,N-Bis-(2-hydroxyethyl)-p-phenylendiamin, 2-Methoxymethyl-p-phenylendiamin, N-(4-Amino-3-methylphenyl)-N-[3-(1H-imidazol-1-yl)propyl]amin, N,N'-Bis-(2-hydroxyethyl)-N,N'-bis-(4-aminophenyl)-1,3-diamino-propan-2-ol, Bis-(2-hydroxy-5-aminophenyl)methan, 1,3-Bis-(2,5-diaminophenoxy)-propan-2-ol, N,N'-Bis-(4-aminophenyl)-1,4-diazacycloheptan, 1,10-Bis-(2,5-diaminophenyl)-1,4,7,10-tetraoxadecan, p-Aminophenol, 4-Amino-3-methylphenol, 4-Amino-2-aminomethylphenol, 4-Amino-2-(1,2-dihydroxyethyl)phenol und 4-Amino-2-(diethylaminomethyl)phenol, 4,5-Diamino-1-(2-hydroxyethyl)pyrazol, 2,4,5,6-Tetraaminopyrimidin, 4-Hydroxy-2,5,6-triaminopyrimidin, 2-Hydroxy-4,5,6-triaminopyrimidin, sowie den physiologisch verträglichen Salzen dieser Verbindungen. Ganz besonders bevorzugte Entwicklerkomponenten sind p-Toluylendiamin, 2-(2-Hydroxyethyl)-p-phenylendiamin, 2-Methoxymethyl-p-phenylendiamin, N-(4-Amino-3-methylphenyl)-N-[3-(1H-imidazol-1-yl)propyl]amin, und/oder 4,5-Diamino-1-(2-hydroxyethyl)pyrazol sowie deren physiologisch verträglichen Salze. Die Entwicklerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Kupplerkomponenten bilden im Rahmen der oxidativen Färbung allein keine signifikante Färbung aus, sondern benötigen stets die Gegenwart von Entwicklerkomponenten. Daher ist es erfindungsgemäß bevorzugt, dass bei Verwendung mindestens einer Kupplerkomponente zusätzlich mindestens eine Entwicklerkomponente zum Einsatz kommt. Erfindungsgemäße Kupplerkomponenten werden bevorzugt ausgewählt aus m-Aminophenol und/oder dessen Derivaten, m-Diaminobenzol und/oder dessen Derivaten, o-Diaminobenzol und/oder dessen Derivaten, o-Aminophenol und/oder dessen Derivaten, Naphthalinderivaten mit mindestens einer Hydroxygruppe, Di- beziehungsweise Trihydroxybenzol und/oder deren Derivaten, Pyridinderivaten, Pyrimidinderivaten, Monohydroxyindol-Derivaten und/oder Monoaminoindol-Derivaten, Monohydroxyindolin-Derivaten und/oder Monoaminoindolin-Derivaten, Pyrazolonderivaten, wie beispielsweise 1-Phenyl-3-methylpyrazol-5-on, Morpholinderivaten, wie beispielsweise 6-Hydroxybenzomorpholin oder 6-Aminobenzomorpholin, Chinoxalinderivaten, wie beispielsweise 6-Methyl-1,2,3,4-tetrahydrochinoxalin, und/oder Gemischen aus zwei oder mehreren Verbindungen aus einer oder mehreren dieser Klassen.

Die erfindungsgemäß verwendbaren m-Aminophenole bzw. deren Derivate werden bevorzugt ausgewählt aus mindestens einer Verbindung aus der Gruppe, die gebildet wird aus 3-Aminophenol, 5-Amino-2-methylphenol, N-Cyclopentyl-3-aminophenol, 3-Amino-2-chlor-6-methylphenol, 2-Hydroxy-4-aminophenoxyethanol, 2,6-Dimethyl-3-aminophenol, 3-Trifluoroacetylamino-2-chlor-6-methylphenol, 5-Amino-4-chlor-2-methylphenol, 5-Amino-4-methoxy-2-methylphenol, 5-(2'-Hydroxyethyl)amino-2-methylphenol, 3-Diethylaminophenol, N-Cyclopentyl-3-aminophenol, 1,3-Dihydroxy-5-(methylamino)benzol, 3-Ethylamino-4-methylphenol, 2,4-Dichlor-3-aminophenol und deren physiologisch verträglichen Salzen.

Die erfindungsgemäß verwendbaren 3-Diaminobenzole bzw. deren Derivate werden bevorzugt ausgewählt aus mindestens einer Verbindung aus der Gruppe, die gebildet wird aus m-Phenylendiamin, 2-(2,4-Diaminophenoxy)ethanol, 1,3-Bis(2,4-diaminophenoxy)propan, 1-Methoxy-2-amino-4-(2'-hydroxyethylamino)benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}-amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2'-hydroxyethyl)aminobenzol und deren physiologisch verträglichen Salzen. Die erfindungsgemäß verwendbaren o-Diaminobenzole bzw. deren Derivate werden bevorzugt ausgewählt aus mindestens einer Verbindung aus der Gruppe, die gebildet wird aus 3,4-Diaminobenzoesäure und 2,3-Diamino-1-methylbenzol und deren physiologisch verträglichen Salzen.

Bevorzugte Di- beziehungsweise Trihydroxybenzole und deren Derivate werden ausgewählt aus mindestens einer Verbindung der Gruppe, die gebildet wird aus Resorcin, Resorcinmonomethylether, 2-Methylresorcin, 5-Methylresorcin, 2,5-Dimethylresorcin, 2-Chlorresorcin, 4-Chlorresorcin, Pyrogallol und 1,2,4-Trihydroxybenzol.

Die erfindungsgemäß verwendbaren Pyridinderivate werden bevorzugt ausgewählt aus mindestens einer Verbindung der Gruppe, die gebildet wird aus 2,6-Dihydroxypyridin, 2-Amino-3-hydroxypyridin, 2-Amino-5-chlor-3-hydroxypyridin, 3-Amino-2-methylamino-6-methoxypyridin, 2,6-Dihydroxy-3,4-dimethylpyridin, 2,6-Dihydroxy-4-methylpyridin, 2,6-Diaminopyridin, 2,3-Diamino-6-methoxypyridin, 3,5-Diamino-2,6-dimethoxypyridin, 3,4-Diaminopyridin, 2-(2-Methoxyethyl)amino-3-amino-6-methoxypyridin, 2-(4'-Methoxyphenyl)amino-3-aminopyridin und deren physiologisch verträglichen Salzen.

Bevorzugte Naphthalinderivate mit mindestens einer Hydroxygruppe werden ausgewählt aus mindestens einer Verbindung der Gruppe, die gebildet wird aus 1-Naphthol, 2-Methyl-1-naphthol, 2-Hydroxymethyl-1-naphthol, 2-Hydroxyethyl-1-naphthol, 1,3-Dihydroxynaphthalin, 1,5-Dihydroxynaphthalin, 1,6-Dihydroxynaphthalin, 1,7-Dihydroxynaphthalin, 1,8-Dihydroxynaphthalin, 2,7-Dihydroxynaphthalin und 2,3-Dihydroxynaphthalin.

Die erfindungsgemäß verwendbaren Indolderivate werden bevorzugt ausgewählt aus 4-Hydroxyindol, 6-Hydroxyindol und 7-Hydroxyindol und deren physiologisch verträglichen Salzen.

Die erfindungsgemäß verwendbaren Indolinderivate werden bevorzugt ausgewählt aus 4-Hydroxyindolin, 6-Hydroxyindolin und 7-Hydroxyindolin und deren physiologisch verträglichen Salzen.

Bevorzugte Pyrimidinderivate werden ausgewählt aus mindestens einer Verbindung der Gruppe, die gebildet wird aus 4,6-Diaminopyrimidin, 4-Amino-2,6-dihydroxypyrimidin, 2,4-Diamino-6-hydroxypyrimidin, 2,4,6-Trihydroxypyrimidin, 2-Amino-4-methylpyrimidin, 2-Amino-4-hydroxy-6-methylpyrimidin und 4,6-Dihydroxy-2-methylpyrimidin und deren physiologisch verträglichen Salzen.

Erfindungsgemäß besonders bevorzugte Kupplerkomponenten werden ausgewählt unter 3-Aminophenol, 5-Amino-2-methylphenol, 3-Amino-2-chlor-6-methylphenol, 2-Hydroxy-4-aminophenoxyethanol, 5-Amino-4-chlor-2-methylphenol, 5-(2-Hydroxyethyl)-amino-2-methylphenol, 2,4-Dichlor-3-aminophenol, 2-Aminophenol, 3-Phenylendiamin, 2-(2,4-Diaminophenoxy)-ethanol, 1,3-Bis(2,4-diaminophenoxy)propan, 1-Methoxy-2-amino-4-(2-hydroxyethylamino)-benzol, 1,3-Bis(2,4-diaminophenyl)propan, 2,6-Bis(2'-hydroxyethylamino)-1-methylbenzol, 2-({3-[(2-Hydroxyethyl)amino]-4-methoxy-5-methylphenyl}amino)ethanol, 2-({3-[(2-Hydroxyethyl)amino]-2-methoxy-5-methylphenyl}amino)ethanol, 2-({3-[(2-Hydroxyethyl)amino]-4,5-dimethylphenyl}amino)ethanol, 2-[3-Morpholin-4-ylphenyl)amino]ethanol, 3-Amino-4-(2-methoxyethoxy)-5-methylphenylamin, 1-Amino-3-bis-(2-hydroxyethyl)aminobenzol, Resorcin, 2-Methylresorcin, 4-Chlorresorcin, 1,2,4-Trihydroxybenzol, 2-Amino-3-hydroxypyridin, 3-Amino-2-methylamino-6-methoxypyridin, 2,6-Dihydroxy-3,4-dimethylpyridin, 3,5-Diamino-2,6-dimethoxypyridin, 1-Phenyl-3-methylpyrazol-5-on, 1-Naphthol, 1,5-Dihydroxynaphthalin, 2,7-Dihydroxynaphthalin, 1,7-Dihydroxynaphthalin, 1,8-Dihydroxynaphthalin, 4-Hydroxyindol, 6-Hydroxyindol, 7-Hydroxyindol, 4-Hydroxyindolin, 6-Hydroxyindolin, 7-Hydroxyindolin oder Gemischen dieser Verbindungen oder deren physiologisch verträglichen Salzen. Besonders bevorzugt sind dabei Resorcin, 2-Methylresorcin, 5-Amino-2-methylphenol, 3-Aminophenol, 2-(2,4-Diaminophenoxy)ethanol, 1,3-Bis-(2,4-diaminophenoxy)propan, 1-Methoxy-2-amino-4-(2'-hydroxyethylamino)benzol, 2-Amino-3-hydroxypyridin und 1-Naphthol sowie eines deren physiologisch verträglichen Salze. Die Kupplerkomponenten werden bevorzugt in einer Menge von 0,0001 bis 10 Gew.-%, vorzugsweise 0,001 bis 5 Gew.-%, jeweils bezogen auf das anwendungsbereite Mittel, verwendet.

Weiterhin können die Mittel zur erfindungsgemäßen Verwendung mindestens einen direktziehenden Farbstoff enthalten. Dabei handelt sich um Farbstoffe, die direkt auf das Haar aufziehen und keinen oxidativen Prozess zur Ausbildung der Farbe benötigen. Direktziehende Farbstoffe sind üblicherweise Nitrophenylendiamine, Nitroaminophenole, Azofarbstoffe, Anthrachinone oder Indophenole. Direktziehende Farbstoffe können in anionische, kationische und nichtionische direktziehende Farbstoffe unterteilt werden. Die direktziehenden Farbstoffe werden jeweils bevorzugt in einer Menge von 0,001 bis 20 Gew.-%, insbesondere von 0,05 bis 5 Gew.-%, jeweils bezogen auf die gesamte Anwendungszubereitung, eingesetzt. Die Gesamtmenge an direktziehenden Farbstoffen beträgt vorzugsweise höchstens 20 Gew.-%.

Bevorzugte anionische direktziehende Farbstoffe sind die unter den Bezeichnungen Acid Yellow 1, Yellow 10, Acid Yellow 23, Acid Yellow 36, Acid Orange 7, Acid Red 33, Acid Red 52, Pigment Red 57:1, Acid Blue 7, Acid Green 50, Acid Violet 43, Acid Black 1, Acid Black 52 und Tetrabromphenolblau bekannten Verbindungen.

Bevorzugte kationische direktziehende Farbstoffe sind dabei kationische Triphenylmethanfarbstoffe, wie Basic Blue 7, Basic Blue 26, Basic Violet 2 und Basic Violet 14, aromatischen Systeme, die mit einer quaternären Stickstoffgruppe substituiert sind, wie Basic Yellow 57, Basic Red 76, Basic Blue 99, Basic Brown 16 und Basic Brown 17 und HC Blue 16, sowie Basic Yellow 87, Basic Orange 31 und Basic Red 51.

Als nichtionische direktziehende Farbstoffe eignen sich insbesondere nichtionische Nitro- und Chinonfarbstoffe und neutrale Azofarbstoffe. Bevorzugte nichtionische direktziehende Farbstoffe sind HC Yellow 2, HC Yellow 4, HC Yellow 5, HC Yellow 6, HC Yellow 12, HC Orange 1, Disperse Orange 3, HC Red 1, HC Red 3, HC Red 10, HC Red 11, HC Red 13, HC Red BN, HC Blue 2, HC Blue 11, HC Blue 12, Disperse Blue 3, HC Violet 1, Disperse Violet 1, Disperse Violet 4, Disperse Black 9, sowie 1,4-Diamino-2-nitrobenzol, 2-Amino-4-nitrophenol, 1,4-Bis-(2-hydroxyethyl)amino-2-nitrobenzol, 3-Nitro-4-(2-hydroxyethyl)aminophenol, 2-(2-Hydroxyethyl)amino-4,6-dinitrophenol, 4-[(2-Hydroxyethyl)amino]-3-nitro-1-methylbenzol, 1-Amino-4-(2-hydroxyethyl)amino-5-chlor-2-nitrobenzol, 4-Amino-3-nitrophenol, 1-(2'-Ureidoethyl)amino-4-nitrobenzol, 2-[(4-Amino-2-nitrophenyl)amino]benzoesäure, 6-Nitro-1,2,3,4-tetrahydrochinoxalin, 2-Hydroxy-1,4-naphthochinon, Pikraminsäure und deren Salze, 2-Amino-6-chloro-4-nitrophenol, 4-Ethylamino-3-nitrobenzoesäure und 2-Chlor-6-ethylamino-4-nitrophenol.

Es ist nicht erforderlich, dass die fakultativ enthaltenen direktziehenden Farbstoffe jeweils einheitliche Verbindungen darstellen. Vielmehr können, bedingt durch die Herstellungsverfahren für die einzelnen Farbstoffe, in untergeordneten Mengen noch weitere Komponenten enthalten sein, soweit diese nicht das Färbeergebnis nachteilig beeinflussen oder aus anderen Gründen, z.B. toxikologischen, ausgeschlossen werden müssen.

Weiterhin können als direktziehende Farbstoffe auch in der Natur vorkommende Farbstoffe eingesetzt werden, wie sie beispielsweise in Henna rot, Henna neutral, Henna schwarz, Kamillenblüte, Sandelholz, schwarzem Tee, Walnuss, Faulbaumrinde, Salbei, Blauholz, Krappwurzel, Catechu und Alkannawurzel enthalten sind.

Als Farbstoffvorstufen naturanaloger Farbstoffe werden bevorzugt solche Indole und Indoline eingesetzt, die mindestens zwei Gruppen ausgewählt aus Hydroxy- und/oder oder Aminogruppen, bevorzugt als Substituent am Sechsring, aufweisen. Diese Gruppen können weitere Substituenten tragen, z. B. in Form einer Veretherung oder Veresterung der Hydroxygruppe oder eine Alkylierung der Aminogruppe. In einer weiteren Ausführungsform enthalten die Färbemittel mindestens ein Indol- und/oder Indolinderivat. Erfindungsgemäße Zusammensetzungen, die Vorstufen naturanaloger Farbstoffe enthalten, werden bevorzugt als luftoxidative Färbemittel verwendet. In dieser Ausführungsform werden die besagten Zusammensetzungen folglich nicht mit einem zusätzlichen Oxidationsmittel versetzt. Die Farbstoffvorstufen naturanaloger Farbstoffe werden jeweils bevorzugt in einer Menge von 0,001 bis 5 Gew.-%, bezogen auf die gesamte Anwendungszubereitung, eingesetzt.

Besonders geeignet als Vorstufen naturanaloger Haarfarbstoffe geeignet sind Derivate des 5,6-Dihydroxyindolins, insbesondere 5,6-Dihydroxyindolin, N-Methyl-5,6-dihydroxyindolin, N-Ethyl-5,6-dihydroxyindolin, N-Propyl-5,6-dihydroxyindolin, N-Butyl-5,6-dihydroxyindolin und/oder 5,6-Dihydroxyindolin-2-carbonsäure, insbesondere bevorzugt 5,6-Dihydroxyindolin. Als Vorstufen naturanaloger Haarfarbstoffe hervorragend geeignet sind weiterhin Derivate des 5,6-Dihydroxyindols , insbesondere 5,6-Dihydroxyindol, N-Methyl-5,6-dihydroxyindol, N-Ethyl-5,6-dihydroxyindol, N-Propyl-5,6-dihydroxyindol, N-Butyl-5,6-dihydroxyindol, 5,6-Dihydroxyindol-2-carbonsäure, insbesondere bevorzugt 5,6-Dihydroxyindol.

Unter Berücksichtigung der bisher genannten bevorzugten Ausführungsformen stellt es eine spezielle und ausdrücklich bevorzugte Ausführungsform dar, wenn das Mittel zum Färben von keratinischen Fasern frei von Ammoniak ist und in einem kosmetischen Träger eine Kombination aus Monoethanolamin und 2-Amino-2-methylpropanol sowie weiterhin mindestens ein Oxidationsfarbstoffprodukt, ausgewählt aus p-Toluylendiamin, 2-(2-Hydroxyethyl)-p-phenylendiamin, 2-Methoxymethyl-p-phenylendiamin, N-(4-Amino-3-methylphenyl)-N-[3-(1 H-imidazol-1-yl)propyl]amin, und/oder 4,5-Diamino-1-(2-hydroxyethyl)pyrazol sowie deren physiologisch verträglichen Salzen, und als dritte Komponente Wasserstoffperoxid in den bereits beschriebenen bevorzugten Mengenanteilen enthält.

Ganz besonders bevorzugt sind schließlich Mittel, die frei von Ammoniak sind und die die nachfolgende Kombination enthalten, worin sich die Gewichtsangaben wiederum auf das Gesamtgewicht des anwendungsbereiten Mittels beziehen:
2,0 bis 6,0 Gew.-% Monoethanolamin,
2,0 bis 6,0 Gew.-% 2-Amino-2-methylpropanol
und 3,0 bis 12,0 Gew.-% Wasserstoffperoxid.

Weiterhin ganz besonders bevorzugt sind schließlich Mittel, die frei von Ammoniak sind und die die nachfolgende Kombination enthalten, worin sich die Gewichtsangaben wiederum auf das Gesamtgewicht des anwendungsbereiten Mittels beziehen:
2,0 bis 6,0 Gew.-% Monoethanolamin,
2,0 bis 6,0 Gew.-% 2-Amino-2-methylpropanol,
0,1 bis 5,0 Gew.-% an Oxidationsfarbstoffvorprodukten
und 3,0 bis 12,0 Gew.-% Wasserstoffperoxid.

Es hat im Laufe der Untersuchungen dieser Erfindung herausgestellt, dass der Zusatz bestimmter Fettkörper eine weitere Verbesserung der Aufhellleistung und des Grauabdeckungsvermögens bewirken kann.

Bevorzugte Fettkörper werden dabei ausgewählt unter Fettalkoholen, Fettsäureester mit Mono- und polyhydroxylierten Alkoholen sowie Estern von Fettalkoholen mit kurzkettigen Mono- und Dicarbonsäuren mit C₂-C₆-Grundkörper.

Besonders bevorzugte Fettkörper sind dabei die Fettsäureester von Fettalkholen, wobei Fettsäure und Fettalkohol gesättigte oder ungesättigt sein können und bevorzugt 8 bis 30, weiter bevorzugt 8 bis 22 Kohlenstoffatome in der Kette tragen.

Eine Ausführungsform der vorliegenden Erfindung ist dadurch gekennzeichnet, dass das Mittel zusätzlich mindestens eine Fettkomponente enthält, die ausgewählt ist aus Fettsäurealkylestern der Formel RCO₂R' worin R für eine C₇-C₂₁-Alkylgruppe oder C₇-C₂₁-Alkenylgruppe und R' für eine C₈-C₂₂-Alkylgruppe oder C₈-C₂₂-Alkenylgruppe stehen.

Erfindungsgemäß bevorzugte Fettsäurealkylester werden ausgewählt aus Decyllaurat, Decylmyristat, Decylpalmitat, Decylstearat, Decyloleat, Lauryllaurat, Laurylmyristat, Laurylpalmitat, Laurylstearat, Lauryloleat, Myristyllaurat, Myristylmyristat, Myristylpalmitat, Myristylstearat, Myristyloleat, Cetyllaurat, Cetylmyristat, Cetylpalmitat, Cetylstearat, Cetyloleat, Stearyllaurat, Stearylmyristat, Stearylpalmitat, Stearylstearat, Stearyloleat, Oleyllaurat, Oleylmyristat, Oleylpalmitat, Oleylstearat, Oleyloleat sowie Mischungen davon. Besonders bevorzugt enthalten die erfindungsgemäßen Mittel zusätzlich Decyloleat (Handelsnamen z. B. Cetiol V, Saboderm) und/oder Oleyloleat (Handelsnamen z. B. Cetiol, Edenor ODO).

Die erfindungsgemäßen Mittel enthalten die Fettsäurealkylestern der Formel RCO₂R' in einer Ausführungsform der vorliegenden Erfindung in einem Gesamtgewicht von 0,5 bis 10 Gew.-%, bevorzugt 1 bis 5 Gew.-%, jeweils bezogen auf das Gesamtgewicht des erfindungsgemäßen Mittels.

Erfindungsgemäß kann ein Oxidationsfärbemittel oder ein oxidatives Aufhellmittel auch zusammen mit einem Katalysator auf das Haar aufgebracht werden, der die Oxidation der Farbstoffvorprodukte, z.B. durch Luftsauerstoff, aktiviert. Solche Katalysatoren sind z. B. bestimmte Enzyme, Iodide, Chinone oder Metallionen.

Weiterhin hat es sich als vorteilhaft erweisen, wenn die Färbe- und/oder Aufhellmittel mindestens einen Stabilisator oder Komplexbildner enthalten. Gebräuchliche und im Rahmen der vorliegenden Erfindung bevorzugte Chelatkomplexbildner sind beispielsweise Polycarbonsäuren, stickstoffhaltige Mono- oder Polycarbonsäuren, insbesondere Ethylendiamintetraessigsäure (EDTA), Ethylendiamindibernsteinsäure (EDDS) und Nitrilotriessigsäure (NTA), geminale Diphosphonsäuren, insbesondere 1-Hydroxyethan-1,1-diphosphonsäure (HEDP), Aminophosphonsäuren wie Ethylendiamintetra(methylenphosphonsäure) (EDTMP), Diethylentriaminpenta(methylenphosphonsäure) (DTPMP), Phosphonopolycarbonsäuren wie 2-Phosphonobutan-1,2,4-tricarbonsäure sowie Cyclodextrine, Alkalistannate (Natriumstannat), Alkalipyrophosphate (Tetranatriumpyrophosphat, Dinatriumpyrophosphat), Alkaliphosphate (Natriumphosphat), und Phosphorsäure. Erfindungsgemäß bevorzugt enthalten die Mittel Komplexbildner zu 0,01 bis 3 Gew.-%, bevorzugt 0,05 bis 1 Gew.-%, jeweils bezogen auf das Gesamtgewicht des erfindungsgemäßen Mittels.

Bei einer Anwendung von zusätzlichen Oxidationsmitteln wird die eigentliche Aufhell- oder Färbezubereitung zweckmäßigerweise unmittelbar vor der Anwendung durch Mischung einer erfindungsgemäßen Zubereitung, enthaltend mindestens eine Kombination aus mindestens einem Alkanolamin gemäß Formel (I) und mindestens einem Alkanolamin gemäß Formel (II), sowie einer Zubereitung, enthaltend das Oxidationsmittel, ausgewählt aus Wasserstoffperoxid und/oder dessen feste Anlagerungsprodukte an anorganische oder organische Verbindungen, hergestellt.

Die erfindungsgemäß verwendbaren Mittel können zusätzlich Blondier- und/oder Bleichmittel enthalten und somit als Mittel bereitgestellt werden, die gleichzeitig färbend und aufhellend wirken. Solche Mittel werden nachfolgend als "Färbemittel", als "aufhellende Färbemittel" oder als "Färbe- und Aufhellmittel" bezeichnet. Für die starke Aufhellung sehr dunklen Haares ist jedoch der alleinige Einsatz von Wasserstoffperoxid oder dessen Anlagerungsprodukten an organische beziehungsweise anorganische Verbindungen oftmals nicht ausreichend.

Daher kann es erfindungsgemäß, sollte der Verbraucher den Wunsch nach einer sehr starken Blondierung verspüren, in einer weiteren Ausführungsform bevorzugt sein, wenn das Färbemittel zusätzlich mindestens ein anorganisches Persulfatsalz bzw. Peroxodisulfatsalz in dem Mittel zum Aufhellen der keratinischen Fasern enthält. Bevorzugte Peroxodisulfatsalze sind Ammoniumperoxodisulfat, Kaliumperoxodisulfat und Natriumperoxodisulfat. Die Peroxodisulfatsalze können in einer Menge von 0,1 bis 25 Gew.-%, insbesondere in einer Menge von 0,5 bis 15 Gew.-%, bezogen auf das Gesamtgewicht des anwendungsbereiten Mittels, enthalten sein.

Die erfindungsgemäß verwendbaren Mittel werden bevorzugt als fließfähige Zubereitungen formuliert. Dazu gehören insbesondere Emulsionen, Suspensionen und Gele, besonders bevorzugt Emulsionen. Bevorzugt enthalten die fließfähigen Zubereitungen zusätzlich als oberflächenaktive Substanz ein Emulgator bzw. ein Tensid, wobei oberflächenaktive Substanzen je nach Anwendungsgebiet als Tenside oder als Emulgatoren bezeichnet werden und aus anionischen, kationischen, amphoteren, zwitterionischen und nichtionischen Tensiden ausgewählt sind.

Als anionische Tenside eignen sich in erfindungsgemäßen Zubereitungen alle für die Verwendung am menschlichen Körper geeigneten anionischen oberflächenaktiven Stoffe. Bevorzugte anionische Tenside sind lineare und verzweigte Fettsäuren mit 8 bis 30 C-Atomen (Seifen), Alkylsulfate, Alkylethersulfate und polyethoxylierte Ethercarbonsäuren mit 10 bis 18 C-Atomen in der Alkylgruppe und bis zu 12 Glykolethergruppen im Molekül.

Als zwitterionische Tenside werden solche oberflächenaktiven Verbindungen bezeichnet, die im Molekül mindestens eine quartäre Ammoniumgruppe und mindestens eine Carboxylat-, Sulfonat- oder Sulfat-Gruppe tragen. Besonders geeignete zwitterionische Tenside sind Betaine. Ein bevorzugtes zwitterionisches Tensid ist das unter der INCI-Bezeichnung Cocamidopropyl Betaine bekannte Fettsäureamid-Derivat.

In einer weiteren Ausführungsform der vorliegenden Erfindung enthält das Mittel weiterhin mindestens ein amphoteres Tensid. Beispiele für geeignete amphotere Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren. Besonders bevorzugte amphotere Tenside werden unter der INCI-Bezeichnung Disodium Cocoamphodipropionate und Disodium Cocoamphodiacetate vermarktet.

Als nichtionische Tenside eignen sich Alkylpolyglykoside, insbesondere C₈-C₂₂-Alkylmono- und -oligoglycoside und deren ethoxylierte Analoga. Als weitere bevorzugte nichtionische Tenside haben sich die Alkylenoxid-Anlagerungsprodukte an gesättigte lineare Fettalkohole und Fettsäuren mit jeweils 2 bis 30 Mol Ethylenoxid pro Mol Fettalkohol bzw. Fettsäure erwiesen. Zubereitungen mit hervorragenden Eigenschaften werden ebenfalls erhalten, wenn sie als nichtionische Tenside Fettsäureester von ethoxyliertem Glycerin enthalten.

Erfindungsgemäß bevorzugt sind ebenfalls kationische Tenside vom Typ der quartären Ammoniumverbindungen, der Esterquats und der Alkylamidoamine. Bevorzugte quaternäre Ammoniumverbindungen sind Ammoniumhalogenide, wie Alkyltrimethylammoniumchloride, Dialkyldimethylammoniumchloride und Trialkylmethylammoniumchloride, sowie die unter den INCI-Bezeichnungen Quaternium-27 und Quaternium-83 bekannten Imidazolium-Verbindungen. Weitere erfindungsgemäß verwendbare kationische Tenside sind quaternisierte Proteinhydrolysate. Alkylamidoamine werden üblicherweise durch Amidierung natürlicher oder synthetischer Fettsäuren und Fettsäureschnitte mit Dialkylaminoaminen hergestellt, Tegoamid^{®} S 18 (Stearamidopropyldimethylamin). Bevorzugte Esterquats sind quaternierte Estersalze von Fettsäuren mit Triethanolamin, quaternierte Estersalze von Fettsäuren mit Diethanolalkylaminen und quaternierten Estersalzen von Fettsäuren mit 1,2-Dihydroxypropyldialkylaminen.

Die anionischen, nichtionischen, amphoteren oder zwitterionischen Tenside werden in Gesamtmengen von 0,1 bis 45 Gew.%, bevorzugt 1 bis 30 Gew.% und besonders bevorzugt von 1 bis 15 Gew.%, bezogen auf die Gesamtmenge des anwendungsbereiten Mittels, eingesetzt. Die kationischen Tenside sind in den erfindungsgemäß verwendeten Mitteln bevorzugt in Mengen von 0,05 bis 10 Gew.-%, besonders bevorzugt 0,1 bis 5 Gew.-%, bezogen auf das gesamte Mittel, enthalten.

Ferner können die erfindungsgemäßen Mittel weitere Wirk-, Hilfs- und Zusatzstoffe enthalten, wie beispielsweise kationische Polymere, nichtionische Polymere (Vinylpyrrolidinon/Vinylacrylat-Copolymere, Polyvinylpyrrolidinon, Vinylpyrrolidinon/Vinylacetat-Copolymere, Polyethylenglykole und Polysiloxane); zwitterionische und amphotere Polymere (Acrylamidopropyl-trimethylammoniumchlorid/Acrylat-Copolymere und Octylacrylamid/Methyl-methacrylat/tert-Butylaminoethylmethacrylat/2-Hydroxypropylmethacrylat-Copolymere); anionische Polymere (Polyacrylsäuren, vernetzte Polyacrylsäuren, Vinylacetat/Crotonsäure-Copolymere, Vinylpyrrolidinon/Vinylacrylat-Copolymere, Vinylacetat/Butylmaleat/Isobornylacrylat-Copolymere, Methylvinylether/Malein-säureanhydrid-Copolymere und Acrylsäure/Ethylacrylat/N-tert-Butylacrylamid-Terpolymere); Verdickungsmittel (Agar-Agar, Guar-Gum, Alginate, Xanthan-Gum, Gummi arabicum, Karaya-Gummi, Johannisbrotkernmehl, Leinsamengummen, Dextrane, Cellulose-Derivate, z. B. Methylcellulose, Hydroxyalkylcellulose und Carboxymethylcellulose, Stärke-Fraktionen und Derivate wie Amylose, Amylopektin und Dextrine, Tone wie z. B. Bentonit oder vollsynthetische Hydrokolloide wie z.B. Polyvinylalkohol); haarkonditionierende Verbindungen (Phospholipide, beispielsweise Sojalecithin, Ei-Lecitin, Kephaline sowie Silikonöle); Proteinhydrolysate pflanzlicher oder tierischer Herkunft (Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate); Parfümöle, Dimethylisosorbid und Cyclodextrine; faserstrukturverbessernde Wirkstoffe (Mono-, Di- und Oligosaccharide, Glucose, Maleinsäure und Milchsäure); Entschäumer wie Silikone (Dimethicon); Farbstoffe zum Anfärben des Mittels; Antischuppenwirkstoffe (Piroctone Olamine, Zink Omadine und Climbazol); Lichtschutzmittel (derivatisierte Benzophenone, Zimtsäure-Derivate und Triazine); Wirkstoffe (Pantolacton, Allantoin, Pyrrolidinoncarbonsäuren und deren Salze sowie Bisabolol); Vitamine, Provitamine und Vitaminvorstufen, insbesondere solche der Gruppen A, B₃, B₅, B₆, C, E, F und H; Pflanzenextrakte; pflanzliche Öle (Macadamianussöl, Kukuinussöl, Palmöl, Amaranthsamenöl, Pfirsichkernöl, Avocadoöl, Olivenöl, Kokosöl, Rapsöl, Sesamöl, Jojobaöl, Sojaöl, Erdnussöl, Nachtkerzenöl und Teebaumöl); Cholesterin; Konsistenzgeber (Zuckerester, Polyolester oder Polyolalkylether); Fette und Wachse (Fettalkohole, Bienenwachs, Montanwachs und Paraffine); Quell- und Penetrationsstoffe (Glycerin, Propylenglykolmonoethylether, Carbonate, Hydrogencarbonate, Guanidine, Harnstoffe sowie primäre, sekundäre und tertiäre Phosphate); Trübungsmittel (Latex, Styrol/PVP- und Styrol/Acrylamid-Copolymere); Perlglanzmittel (Ethylenglykolmono- und -distearat sowie PEG-3-distearat); Treibmittel wie Propan-Butan-Gemische, N₂O, Dimethylether, CO₂ und Luft; Antioxidantien.

Die Auswahl dieser weiteren Stoffe wird der Fachmann gemäß den gewünschten Eigenschaften der Zubereitungen treffen. Die erfindungsgemäß eingesetzten Zubereitungen enthalten die weiteren Wirk-, Hilfs- und Zusatzstoffe bevorzugt in Mengen von 0,01 bis 25 Gew.-%, insbesondere 0,05 bis 15 Gew.-%, bezogen auf die Gesamtmenge des anwendungsbereiten Mittel.

Die Färbezubereitungen der erfindungsgemäßen Verwendung weisen bevorzugt einen pH-Wert im Bereich von 4 bis 12 aufweist. Im Fall von Oxidationsfärbemitteln findet die Anwendung der Färbemittel in einem schwach alkalischen Milieu statt, bevorzugt bei einem pH-Wert im Bereich von 8,0 bis 10,5. Bei den pH-Werten im Sinne der vorliegenden Erfindung handelt es sich um pH-Werte, die bei einer Temperatur von 22°C gemessen wurden.

Zur Einstellung des pH-Werts sind dem Fachmann neben dem möglichst zu vermeidenden Ammoniak und der erfindungsgemäßen Kombination der Strukturell unterschiedlichen Alkanolamine gängige Acidifizierungs- und Alkalisierungsmittel geläufig. Die zur Einstellung des pH-Wertes verwendbaren Alkalisierungsmittel werden typischerweise gewählt aus anorganischen Salzen, insbesondere der Alkali- und Erdalkalimetalle, organischen Alkalisierungsmitteln, insbesondere Aminen und basische Aminosäuren. Erfindungsgemäß bevorzugte Acidifizierungsmittel sind Genuss-Säuren, wie beispielsweise Zitronensäure, Essigsäure, Äpfelsäure oder Weinsäure, sowie verdünnte Mineralsäuren. Erfindungsgemäß einsetzbare, anorganische Alkalisierungsmittel sind bevorzugt ausgewählt aus der Gruppe, die gebildet wird aus Natriumhydroxid, Kaliumhydroxid, Calciumhydroxid, Bariumhydroxid, Natriumphosphat, Kaliumphosphat, Natriumsilicat, Kaliumsilicat, Natriumcarbonat und Kaliumcarbonat. Besonders bevorzugt sind Natriumhydroxid und/oder Kaliumhydroxid. Die basischen Aminosäuren werden bevorzugt ausgewählt aus der Gruppe, die gebildet wird aus L-Arginin, D-Arginin, D/L-Arginin, L-Lysin, D-Lysin, D/L-Lysin, besonders bevorzugt L-Arginin, D-Arginin und D/L-Arginin. Bevorzugt werden zusätzliche Acidificierungsmittel und Alkalisierungsmittel jeweils in einer Menge von 0,05 bis 15 Gew.-%, insbesondere von 0,5 bis 10 Gew.-%, bezogen auf das Gesamtgewicht des anwendungsbereiten Mittels, eingesetzt.

Das anwendungsbereite Färbe- und/oder Aufhellmittel wird auf die keratinischen Fasern aufgetragen und für eine bestimmte Einwirkzeit auf der Faser, insbesondere im Haar belassen. Die Auftragung der Zubereitung erfolgt üblicherweise mit der Hand durch den Anwender. Bevorzugt wird dabei persönliche Schutzkleidung getragen, insbesondere geeignete Schutzhandschuhe, beispielsweise aus Kunststoff oder Latex (Einweghandschuhe). Es ist aber auch möglich, die Zubereitung mit einer Applikationshilfe auf die keratinischen Fasern aufzutragen. Die Auftragungs- und die Einwirktemperatur der Zubereitung beträgt Raumtemperatur bis 45°C. Gegebenenfalls kann die Wirkung der Zubereitung durch externe Wärmezufuhr, wie beispielsweise mittels einer Wärmehaube, verstärkt werden. Die bevorzugte Einwirkdauer der Zubereitung auf die keratinische Faser beträgt 2 bis 60 min, bevorzugt 5 bis 45 min. Nach Beendigung der Einwirkdauer wird das verbliebene Mittel aus den keratinischen Fasern mit Hilfe einer Reinigungszubereitung oder Wasser ausgewaschen. Nach dem Auswaschen werden die keratinischen Fasern gegebenenfalls mit einem Handtuch oder einem Heißluftgebläse getrocknet.

Ein weiterer Gegenstand der vorliegenden Erfindung ist die kosmetische, nicht therapeutische Verwendung eines Mittels des ersten Erfindungsgegenstands zur Verbesserung der Aufhellleistung bei der oxidativen Aufhellung oder Färbung keratinischer Fasern, insbesondere menschlicher Haare

Die erfindungsgemäßen Mittel können direkt vor der Anwendung aus zwei oder mehreren getrennt verpackten Zubereitungen hergestellt werden. Dies bietet sich insbesondere zur Trennung inkompatibler Inhaltsstoffe an, um eine vorzeitige Reaktion zu vermeiden.

Ein weiterer Gegenstand der vorliegenden Erfindung ist eine Mehrkomponentenverpackungseinheit (Kit-of-Parts),
umfassend mindestens einem ersten Container (C1) mit einer Zubereitung (A),
enthaltend in einem kosmetischen Träger mindestens ein Alkanolamin gemäß Formel (I) und mindestens ein Alkanolamin gemäß Formel (II),
und mindestens einen zweiten Container (C2) mit einer Entwicklerzubereitung (B), enthaltend in einem kosmetischen Träger mindestens ein Oxidationsmittel,
welcher dadurch gekennzeichnet ist, dass sowohl Zubereitung (A) als auch Entwicklerzubereitung (B) frei von Ammoniak sind.

Der Begriff "Container" kennzeichnet hierbei eine Aufnahmemöglichkeit, unabhängig von deren Form, Material oder Verschluss, welche die Möglichkeit beinhaltet, Stoffe oder Stoffgemische zu beinhalten. Der Begriff "Container" umfasst daher, ohne darauf beschränkt zu sein, das Innere einer Tube, eines Beutels oder Sackes, eines Kanisters, einer Dose, einer Wanne, einer Flasche, eines Glases oder eines Paketes, eines Kartons, einer Box, eines Umschlages oder eines anderen Behältnisses. Die Komponenten der Färbezubereitung können in einem einzelnen Container enthalten sein, es ist aber auch möglich und gegebenenfalls bevorzugt, sie auf verschiedene Behälter aufzuteilen und den Verbraucher anzuleiten, sie vor der Anwendung miteinander zu mischen.

Eine bevorzugte Ausführungsform des Erfindungsgegenstands ist eine Mehrkomponentenverpackungseinheit, welche dadurch gekennzeichnet ist, dass die Zubereitung (A) mindestens eine farbgebende Komponente, ausgewählt aus mindestens einem Oxidationsfarbstoffvorprodukt und/oder mindestens einem direktziehenden Farbstoff, enthält.

In einer besonders bevorzugten Ausführungsform ist die Verpackungseinheit dadurch gekennzeichnet, dass sie mindestens eine zusätzliche Komponente, ausgewählt aus persönlicher Schutzbekleidung, wie Einweghandschuhen, Schürze, Applikationshilfe, wie Kamm, Bürste, Pinsel oder Applicette, und Gebrauchsanleitung enthält. Die Gebrauchsanleitung enthält insbesondere Informationen und Anweisungen für den Verbraucher (m/f) zur Anwendung der Mittel aus den Behältern der Verpackungseinheit in einem Verfahren gemäß dem ersten Erfindungsgegenstand. Unter einer Applicette wird ein breiter Pinsel verstanden, an dessen Stielende sich eine Spitze befindet, die das Abteilen von Faserbündeln bzw. Strähnchen aus der Gesamtmenge der Fasern erlaubt und vereinfacht.

Das anwendungsbereite Aufhell- und/oder Färbemittel wird durch Vermischen von Zubereitung (A) mit Entwicklerzubereitung (B) der Mehrkomponentenverpackungseinheit hergestellt.

Die bevorzugten Ausführungsformen des ersten Erfindungsgegenstands gelten mutatis mutandis für die erfindungsgemäße Verwendung und Verpackungseinheit der weiteren Erfindungsgegenstände. Die nachfolgenden Beispiele sollen bevorzugte Ausführungsformen der Erfindung erläutern, ohne sie jedoch einzuschränken.

### Beispiele zur Aufhellung und Färbung

### 1.1. Herstellung einer Aufhellcreme

Aus den aufgelisteten Bestandteilen wurden Blondiercremes wie folgt hergestellt:

| **Rohstoff** | **Gew.%** | | | | |
|---|---|---|---|---|---|
| | **E1** | **V1** | **V2** | **V3** | **V4** |
| Ammoniumcarbomer, 1,0% | -- | -- | -- | -- | 15,7 |
| Natriumlaurvlethersulfat | -- | -- | -- | -- | 0,74 |
| Kaliumoleinseife, 12,5% | -- | -- | -- | -- | 3,15 |
| Lanette N | 14,00 | 14,00 | 14,00 | 14,00 | -- |
| Xanthan | 0,05 | 0,05 | 0,05 | 0,05 | -- |
| Cetiol V | 2,30 | 2,30 | 2,30 | 2,30 | -- |
| Cetearyl alcohol | 3,90 | 3,90 | 3,90 | 3,90 | 12,60 |
| Cutina GMS SE | 6,00 | 6,00 | 6,00 | 6,00 | 2,10 |
| Cutina AGS | -- | -- | -- | -- | 2,10 |
| Eutanol G | -- | -- | -- | -- | 2,10 |
| Ceteareth-20 | -- | -- | -- | -- | 3,15 |
| Titandioxid | -- | -- | -- | -- | 0,50 |
| Merquat Plus 3330 | -- | -- | -- | -- | 1,50 |
| Phospholipid EFA | -- | -- | -- | -- | 0,10 |
| Cocosamidopropylbetain, 40% | 2,00 | 2,00 | 2,00 | 2,00 | - |
| Natriumsulfit | 0,30 | 0,30 | 0,30 | 0,30 | - |
| Ascorbinsäure | 0,10 | 0,10 | 0,10 | 0,10 | 0,05 |
| Natriumsilikat 40 / 42 | 0,50 | 0,50 | 0,50 | 0,50 | -- |
| RonaCare Ectoin | 0,20 | 0,20 | 0,20 | 0,20 | -- |
| RonaCare Tiliroside | 1,00 | 1,00 | 1,00 | 1,00 | -- |
| Traubenkernöl | 1,00 | 1,00 | 1,00 | 1,00 | -- |
| EDTA, Na4, 87% | -- | -- | -- | -- | 0,20 |
| Monoethanolamin | 4,00 | 8,00 | 12,00 | - | -- |
| 2-Amino-2-methyl-propanol | 4,00 | -- | -- | 8,00 | -- |
| Ammoniak, 25% | -- | -- | -- | -- | 10,00 |
| Parfum | qs | qs | qs | qs | qs |
| Wasser | ad 100 | ad 100 | ad 100 | ad 100 | ad 100 |

| | |
|---|---|
| Lanette N | INCI-Bezeichnung: Cetearyl alcohol, Sodium Cetearyl Sulfate (Cognis) |
| Cetiol V | INCI-Bezeichnung: Decyl Oleate (Cognis) |
| Cutina GMS SE | INCI-Bezeichnung: Glyceryl Stearate (Cognis) |
| Cutina AGS | INCI-Bezeichnung: Glycol Distearate (Cognis) |
| Eutanol G | INCI-Bezeichnung: Octyldodecanol (Cognis) |
| Merquat Plus 3330 | INCI-Bezeichnung: Polyquaternium-39 (Nalco) |
| Phospholipid EFA | INCI-Bezeichnung: Linoleamidopropyl PG-Dimonium Chloride Phosphate (Uniquema) |
| RonaCare Ection | INCI-Bezeichnung: Ectoin (Rona/Merck KGaA) |
| RonaCare Tiliroside | INCI-Bezeichnung: Sorbitol, Tiliroside (Rona/Merck KGaA) |
| Natriumsilikat 40/42 | Natronwasserglas |

Die Fettstoffe und Tenside wurden zusammen bei 80 °C aufgeschmolzen und mit einem Teil der Wassermenge dispergiert. Anschließend wurden die restlichen Rezepturbestandteile unter Rühren der Reihe nach eingearbeitet. Dann wurde mit Wasser auf 100 Gew.-% aufgefüllt und die Formulierung kalt gerührt.

Bei den Rezepturen V1 bis V3 handelt es sich um nicht eine erfindungsgemäße Vergleichsrezepturen, die Rezeptur E1 ist ein erfindungsgemäßes Beispiel mit der Kombination der strukturell unterschiedlichen Alkanolamine. V4 ist ein übliches, oxidatives Aufhellmittel mit Ammoniak als Alkalisierungsmittel.

### 1.2. Vermischen mit Entwicklerdispersionen und Anwendung

Die Aufhellcremes wurden im Verhältnis 1:1 mit einer wie folgt zusammengesetzten Entwicklerdispersion (Angaben in Gew.-%) ausgemischt.

| **Rohstoff** | **EW1** | **EW2** | **EW3** | **EW4** | **EW5** |
|---|---|---|---|---|---|
| Kaliumhydroxid, 50 % | je 0,12 | | | | |
| Natriumbenzoat | je 0,04 | | | | |
| Dinatriumpyrophosphat | je 0,10 | | | | |
| HEDP, 60% | je 0,25 | | | | |
| Isopropylmyristat | je 0,80 | | | | |
| Cetearyl Alcohol | je 4,00 | | | | |
| Ceteareth-20 | je 1,00 | | | | |
| Bienenwachs | je 0,30 | | | | |
| Wasserstoffperoxid, 50 % | 5,1 | 11,1 | 17,0 | 23,0 | 24,0 |
| Wasser | je ad 100 | | | | |

Zur Anwendung wurde auf Strähnen hellbraunen Haares (Fischbach-Miller) von ca. 0,7 g Gewicht die 4-fache Menge der fertigen Anwendungsmischung appliziert. Nachdem die Strähnen für 45 min bei 35 °C behandelt wurden, wurden sie mit einem handelsüblichen Shampoo gewaschen und mit einem Föhn getrocknet.

### 1.3. Auswertung der Aufhellleistung

Die farbmetrischen Messungen erfolgten auf der Strähne an jeweils 4 Messpunkten. Als Messgerät diente der Spectralflash SF 450 der Firma Datacolor.

Die Ergebnisse der Messungen wurden mithilfe des CIELAB Farbenraums quantifiziert. Der L-Wert steht dabei für die Helligkeit der Färbung (schwarz-weiß-Achse); je größer der Wert für L ist, desto heller ist die Färbung. Je höher der L-Wert ist, desto stärker ist die Aufhellung der jeweiligen Nuance.

Folgende Aufhellergebnisse wurden erhalten:

| Rezeptur | Entwicklerdispersion | pH-Wert | H₂O₂-Gehalt Anwendungsmischung- [%] | ΔL |
|---|---|---|---|---|
| E1 | EW1 | 9,9 | 1,25 | 11,6 |
| E1 | EW2 | 9,9 | 2,75 | 14,4 |
| E1 | EW3 | 9,9 | 4,25 | 14,1 |
| E1 | EW4 | 9,9 | 5,75 | 16,3 |
| V1 | EW4 | 9,2 | 5,75 | 7,2 |
| V2 | EW4 | 9,9 | 5,75 | 15,9 |
| V3 | EW4 | 9,9 | 5,75 | 12,2 |
| V2 | EW5 | 10,0 | 6,00 | 19,9 |

Die erfindungsgemäßen Aufhellmittel (E1) zeigen bereits bei deutlich geringeren Wasserstoffperoxidkonzentrationen eine signifikante Verbesserung der Aufhellleistung (L-Wert) gegenüber den relevanten Vergleichsrezepturen V1 und V3 ohne die erfindungsgemäße Alkanolamin-Kombination. Selbst Mittel mit deutlich höherem Anteil an einem Alkanolamin (V2) erreichen nicht die Aufhellleistung der erfindungsgemäßen Mittel E1.

Mit E1 werden hingegen nahezu die Aufhellwerte erzielt, die sonst nur mit ammoniak-haltigen Mitteln (V4) zu erhalten sind.

### 2.1 Färbemittel

In die Mittel E1 und V1 gemäß 1.1 wurde zusätzlich noch jeweils folgende Färbemischung eingearbeitet: p-Toluylendiaminsulfat (0,30 Gew.-%); Resorcin (0,03 Gew.-%); 2-Methylresorcin (0,07 Gew.%); 4-Chlorresorcin (0,04 Gew.-%); 2-Amino-3-hydroxypyridin (0,03 Gew.-%); 2-Amino-6-chloro4-nitrophenol (0,02 Gew.-%) und 2-(4-Methyl-2-nitrophenyl)aminoethanol (0,02 Gew.-%). Damit wurden die Färbemittel E1* und V1* erhalten.

### 2.2 Ausfärbung/Grauabdeckung

Die Mittel gemäß 2.1 wurden jeweils mit der Entwicklerzubereitung (EW4) versetzt und im Halbseitentest auf dem Haar von Probanden mit mittlerem bis starken Ergrauungsgrad für 45 min bei 35 °C angewendet. Anschließend wurden die Haare ausgewaschen, mit einem Heißluftgebläse getrocknet und die Färbeergebnisse von Fachpersonal visuell begutachtet.

Dabei zeigte sich, dass das erfindungsgemäße Mittel E1* im Vergleich zum Mittel V1* zu einer deutlich verbesserten Grauabdeckung führte.

## Patentansprüche

1. Mittel zur oxidativen Färbung und/oder Aufhellung keratinischer Fasern, insbesondere menschlicher Haare, enthaltend in einem kosmetischen Träger mindestens ein Oxidationsmittel, ausgewählt aus Wasserstoffperoxid und/oder dessen feste Anlagerungsprodukte an anorganische oder organische Verbindungen, mindestens ein Alkanolamin gemäß Formel (I),
NH₃₋ₓ(CH₂CH₂OH)ₓ (I),
worin x für eine der Zahlen 1, 2 oder 3 steht,
und mindestens ein Alkanolamin gemäß Formel (II), 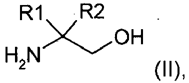 worin R1 und R2 jeweils unabhängig voneinander für Wasserstoff, eine C₁-C₆-Alkylgruppe oder eine C₁-C₆-Hydroxyalkylgruppe stehen oder R1 und R2 zusammen mit dem Kohlenstoffatom des Alkanolamins einen Ring bilden, welcher 4 bis 8 Ringatome besitzt und gegebenenfalls 1 bis 2 Heteroatome enthalten kann, mit der Massgabe, dass R1 und R2 nicht gleichzeitig für Wasserstoff stehen,
**dadurch gekennzeichnet, dass** das Mittel frei von Ammoniak ist und
dass das Mittel Alkanolamine gemäß Formel (I) und Alkanolamine gemäß Formel (II) in einer Gesamtmenge von 0,5 bis 25 Gew.-%, bezogen auf das Gesamtgewicht des anwendungsbereiten Mittels, enthält, wobei das Gewichtsverhältnis zwischen Alkanolaminen gemäß Formel (I) und Alkanolaminen gemäß Formel (II) einen Wert von 1 zu 2 bis 2 zu 1 besitzt.

2. Mittel nach Anspruch 1, **dadurch gekennzeichnet, dass** das Mittel als Alkanolamin gemäß Formel (I) Monoethanolamin enthält.

3. Mittel nach einem der Ansprüche 1 und 2, **dadurch gekennzeichnet, dass** das Mittel als Alkanolamin gemäß Formel (II) mindestens eine Verbindung gemäß Formel (II) enthält, worin R1 und/oder R2 für eine C₁-C₆-Alkylgruppe stehen oder R1 und R2 gemeinsam mit dem Kohlenstoffatom des Alkanolamins für einen Cyclopentyl-Rest, einen Cyclohexyl-Rest oder einen Tetrahydropyran-Rest stehen.

4. Mittel nach einem der Ansprüche 1 bis 3, **dadurch gekennzeichnet, dass** das Mittel als Alkanolamin gemäß Formel (I) Monoethanolamin (x = 1) und als Alkanolamin gemäß Formel (II) mindestens eine Verbindung gemäß Formel (II) enthält, worin R1 und R2 jeweils für eine Methylgruppe stehen.

5. Mittel nach einem der Ansprüche 1 bis 4, **dadurch gekennzeichnet, dass** das Mittel Alkanolamine gemäß Formel (I) und Alkanolamine gemäß Formel (II) in einer Gesamtmenge von 1 bis 20 Gew.-% und besonders bevorzugt von 4 bis 12 Gew.-%, jeweils bezogen auf das Gesamtgewicht des anwendungsbereiten Mittels, enthält, wobei das Gewichtsverhältnis zwischen Alkanolaminen gemäß Formel (I) und Alkanolaminen gemäß Formel (II) einen Wert von 1 zu 2 bis 2 zu 1, besitzt.

6. Mittel nach einem der Ansprüche 1 bis 5, **dadurch gekennzeichnet, dass** das Mittel als farbverändernde Komponente mindestens ein Oxidationsfarbstoffvorprodukt und/oder direktziehenden Farbstoff enthält.

7. Mittel nach einem der Ansprüche 1 bis 6, **dadurch gekennzeichnet, dass** das Mittel zusätzlich mindestens eine Fettkomponente enthält, die ausgewählt ist aus Fettsäurealkylestern der Formel RCO₂R' worin R für eine C₇-C₂₁-Alkylgruppe oder C₇-C₂₁-Alkenylgruppe und R' für eine C₈-C₂₂-Alkylgruppe oder C₈-C₂₂-Alkenylgruppe stehen.

8. Kosmetische, nicht therapeutische Verwendung eines Mittels nach einem der Ansprüche 1 bis 7 zur Verbesserung der Aufhellleistung bei der oxidativen Aufhellung oder Färbung keratinischer Fasern, insbesondere menschlicher Haare.

9. Mehrkomponentenverpackungseinheit (Kit-of-Parts),
umfassend mindestens einen ersten Container (C1) mit einer Zubereitung (A), enthaltend in einem kosmetischen Träger mindestens ein Alkanolamin gemäß Formel (I) und mindestens ein Alkanolamin gemäß Formel (II) wie im Anspruch 1, **dadurch gekennzeichnet, dass** die Zubereitung (A) Alkanolamine gemäß Formel (I) und Alkanolamine gemäß Formel (II) in einer Gesamtmenge von 0,5 bis 25 Gew.-%, bezogen auf das Gesamtgewicht des anwendungsbereiten Mittels, enthält, wobei das Gewichtsverhältnis zwischen Alkanolaminen gemäß Formel (I) und Alkanolaminen gemäß Formel (II) einen Wert von 1 zu 2 bis 2 zu 1 besitzt,
und mindestens einen zweiten Container (C2) mit einer Entwicklerzubereitung (B), enthaltend in einem kosmetischen Träger mindestens ein Oxidationsmittel,
**dadurch gekennzeichnet, dass** sowohl Zubereitung (A) als auch Entwicklerzubereitung (B) frei von Ammoniak sind.

10. Mehrkomponentenverpackungseinheit (Kit-of-Parts) gemäß Anspruch 9, **dadurch gekennzeichnet, dass** die Zubereitung (A) mindestens eine farbgebende Komponente, ausgewählt aus mindestens einem Oxidationsfarbstoffvorprodukt und/oder mindestens einem direktziehenden Farbstoff, enthält.

## Claims

1. An agent for oxidatively dyeing and/or lightening keratinic fibers, in particular human hair, containing, in a cosmetic carrier, at least one oxidizing agent selected from hydrogen peroxide and/or its solid addition products with inorganic or organic compounds, at least one alkanolamine according to formula (I),
NH₃₋ₓ(CH₂CH₂OH)ₓ (I),
in which x stands for one of the numbers 1, 2 or 3,
and at least one alkanolamine according to formula (II), 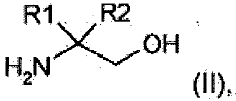 in which R1 and R2, independently of one another, each stand for hydrogen, a C₁-C₆ alkyl group or a C₁-C₆ hydroxyalkyl group, or R1 and R2 together with the carbon atom of the alkanolamine form a ring that contains 4 to 8 ring atoms and can optionally contain 1 to 2 hetero atoms, provided that R1 and R2 do not simultaneously stand for hydrogen, **characterized in that** the agent is free of ammonia, and
**in that** the agent contains alkanolamines according to formula (I) and alkanolamines according to formula (II) in a total amount of from 0.5 to 25 wt.% based on the total weight of the ready-to-use agent, the weight ratio between alkanolamines according to formula (I) and alkanolamines according to formula (II) being from 1 to 2 to 2 to 1.

2. The agent according to claim 1, **characterized in that** the agent contains monoethanolamine as the alkanolamine according to formula (I).

3. The agent according to either claim 1 or claim 2, **characterized in that** the agent contains at least one compound according to formula (II) as the alkanolamine according to formula (II), in which R1 and/or R2 stands for a C₁-C₆ alkyl group, or R1 and R2 together with the carbon atom of the alkanolamine stand for a cyclopentyl functional group, a cyclohexyl functional group or a tetrahydropyran functional group.

4. The agent according to one of claims 1 to 3, **characterized in that** the agent contains monoethanolamine (x = 1) as the alkanolamine according to formula (I) and at least one compound according to formula (II) as the alkanolamine according to formula (II), in which R1 and R2 each stand for a methyl group.

5. The agent according to one of claims 1 to 4, **characterized in that** the agent contains alkanolamines according to formula (I) and alkanolamines according to formula (II) in a total amount of from 1 to 20 wt.%, and particularly preferably from 4 to 12 wt.%, based on the total weight of the ready-to-use agent in each case, the weight ratio between alkanolamines according to formula (I) and alkanolamines according to formula (II) being from 1 to 2 to 2 to 1.

6. The agent according to one of claims 1 to 5, **characterized in that** the agent contains at least one oxidation dye precursor and/or direct dye as the color-changing component.

7. The agent according to one of claims 1 to 6, **characterized in that** the agent additionally contains at least one fatty component selected from fatty acid alkyl esters of formula RCO₂R', in which R stands for a C₇-C₂₁ alkyl group or C₇-C₂₁ alkenyl group and R' stands for a C₈-C₂₂ alkyl group or C₈-C₂₂ alkenyl group.

8. The cosmetic, non-therapeutic use of an agent according to one of claims 1 to 7 for improving the lightening performance when oxidatively lightening or dyeing keratinic fibers, in particular human hair.

9. A kit of parts
comprising at least one first container (C1) having a preparation (A) containing, in a cosmetic carrier, at least one alkanolamine according to formula (I) and at least one alkanolamine according to formula (II) as in claim 1,
**characterized in that** the preparation (A) contains alkanolamines according to formula (I) and alkanolamines according to formula (II) in a total amount of from 0.5 to 25 wt.% based on the total weight of the ready-to-use agent, the weight ratio between alkanolamines according to formula (I) and alkanolamines according to formula (II) being from 1 to 2 to 2 to 1,
and at least one second container (C2) having a developer preparation (B) containing, in a cosmetic carrier, at least one oxidizing agent,
**characterized in that** both the preparation (A) and the developer preparation (B) are free of ammonia.

10. The kit of parts according to claim 9, **characterized in that** the preparation (A) contains at least one color-imparting component selected from at least one oxidation dye precursor and/or at least one direct dye.

## Revendications

1. Agent de coloration et/ou d'éclaircissement par oxydation de fibres kératiniques, en particulier de cheveux humains contenant, dans un support cosmétique, au moins un agent oxydant choisi parmi le peroxyde d'hydrogène et/ou ses produits d'addition solides sur des composés inorganiques ou organiques, au moins un alcool aminé selon la formule (I),
NH₃₋ₓ(CH₂CH₂OH)ₓ (I),
où x représente un des chiffres 1, 2 ou 3,
et au moins un alcool aminé selon la formule (II), 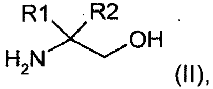 où R1 et R2 représentent respectivement, indépendamment l'un de l'autre, l'hydrogène, un groupe alkyle en C₁-C₆ ou un groupe hydroxyalkyle en C₁-C₆ ou R1 et R2 forment, conjointement avec l'atome de carbone de l'alcool aminé, un cycle qui possède 4 à 8 atomes cycliques et peut contenir le cas échéant 1 à 2 hétéroatomes, sous réserve que R1 et R2 ne représentent pas simultanément l'hydrogène,
**caractérisé en ce que** l'agent est exempt d'ammoniaque et
**en ce que** l'agent contient des alcools aminés selon la formule (I) et des alcools aminés selon la formule (II) dans une quantité totale de 0,5 à 25 % en poids, par rapport au poids total de l'agent prêt à l'emploi, le rapport pondéral entre les alcools aminés selon la formule (I) et les alcools aminés selon la formule (II) possédant une valeur de 1 sur 2 à 2 sur 1.

2. Agent selon la revendication 1 **caractérisé en ce que** l'agent contient de la monoéthanolamine comme alcool aminé selon la formule (I).

3. Agent selon l'une des revendications 1 et 2 **caractérisé en ce que** l'agent contient au moins un composé selon la formule (II) comme alcool aminé selon la formule (II), où R1 et/ou R2 représentent un groupe alkyle en C₁-C₆ ou R1 et R2 représentent conjointement avec l'atome de carbone de l'alcool aminé un radical de cyclopentyle, un radical de cyclohexyle ou un radical de tétrahydropyrane.

4. Agent selon l'une quelconque des revendications 1 à 3 **caractérisé en ce que** l'agent contient de la monoéthanolamine (x = 1) comme alcool aminé selon la formule (I) et au moins un composé selon la formule (II) comme alcool aminé selon la formule (II), où R1 et R2 représentent chacun un groupe méthyle.

5. Agent selon l'une quelconque des revendications 1 à 4 **caractérisé en ce que** l'agent contient des alcools aminés selon la formule (I) et des alcools aminés selon la formule (II) dans une quantité totale de 1 à 20 % en poids et de manière particulièrement préférée de 4 à 12 % en poids, par rapport au poids total de l'agent prêt à l'emploi respectivement, le rapport pondéral entre les alcools aminés selon la formule (I) et les alcools aminés selon la formule (II) possédant une valeur de 1 sur 2 à 2 sur 1.

6. Agent selon l'une quelconque des revendications 1 à 5 **caractérisé en ce que** l'agent contient au moins un précurseur de colorant par oxydation et/ou un colorant à action directe comme constituant de changement de la couleur.

7. Agent selon l'une quelconque des revendications 1 à 6 **caractérisé en ce que** l'agent contient, en plus, au moins un constituant gras qui est choisi parmi des esters d'acide gras et d'alkyle de formule RCO₂R' où R représente un groupe alkyle en C₇-C₂₁ ou un groupe alcényle en C₇-C₂₁, et R', un groupe alkyle en C₈-C₂₂ ou un groupe alcényle en C₈-C₂₂.

8. Utilisation cosmétique non thérapeutique d'un agent selon l'une quelconque des revendications 1 à 7 afin d'améliorer la capacité d'éclaircissement en cas d'éclaircissement ou de coloration de fibres kératiniques par oxydation, notamment de cheveux humains.

9. Unité de conditionnement à composants multiples (Kit-of-Parts),
comprenant au moins un premier contenant (C1) avec une préparation (A), contenant, dans un support cosmétique, au moins un alcool aminé selon la formule (I) et au moins un alcool aminé selon la formule (II) comme dans la revendication 1,
**caractérisée en ce que** la préparation (A) contient des alcools aminés selon la formule (I) et des alcools aminés selon la formule (II) dans une quantité totale de 0,5 à 25 % en poids, par rapport au poids total de l'agent prêt à l'emploi, le rapport pondéral entre les alcools aminés selon la formule (I) et les alcools aminés selon la formule (II) possédant une valeur de 1 sur 2 à 2 sur 1,
et au moins un deuxième contenant (C2) avec une préparation de développeur (B), contenant, dans un support cosmétique, au moins un agent d'oxydation,
**caractérisée en ce que** tant la préparation (A) que la préparation de développeur (B) sont exemptes d'ammoniaque.

10. Unité de conditionnement à composants multiples (Kit-of-Parts) selon la revendication 9, **caractérisée en ce que** la préparation (A) contient au moins un constituant colorant choisi parmi au moins un précurseur de colorant par oxydation et/ou au moins un colorant à action directe.
